# EUROPEAN PATENT APPLICATION

(11) **EP 4 086 847 A1**
(43) Date of publication of application: **09.11.2022**
(21) Application number: 22169766.7
(22) Date of filing: 25.04.2022
(51) Int. Cl.: G06T 7/11, G06T 7/136, G06T 11/00

(54) **METHODS AND SYSTEMS FOR ROBUST INTENSITY RANGES FOR AUTOMATIC LESION VOLUME MEASUREMENT**

(30) Priority: 03.05.2021 US 202117306642
(71) Applicant: GE Precision Healthcare LLC, Wauwatosa, WI 53226 (US)
(72) Inventor: GOGIN, Nicolas, 78530 Buc (FR); COUROT, Adele, 78530 Buc (FR); COUSTAUD, Nicolas, 78530 Buc (FR); AKNOUN, Sherazade, 78530 Buc (FR)
(74) Representative: Fennell, Gareth Charles

(57) **Abstract**

Systems and methods are provided for robust intensity ranges for automatic lesion volume measurement. Imaging data obtained during medical imaging examination of a patient may be processed, with the imaging data corresponding to a particular medical imaging technique. One or more parameters pertinent to identifying particular features in at least one organ or body structure may be automatically determined. At least one medical image may be generated and displayed based on processing of the imaging data. The displaying may include identifying, based on the one or more parameters, one or more areas in the at least one organ or body structure and providing within displayed at least one medical image visual feedback relating to the identified one or more areas in the at least one organ or body structure.

## Description

### FIELD

Aspects of the present disclosure relate to medical imaging solutions. More specifically, certain embodiments relate to methods and systems for robust intensity ranges for automatic lesion volume measurement.

### BACKGROUND

Various medical imaging techniques may be used, such as in imaging organs and soft tissues in a human body. Examples of medical imaging techniques include ultrasound imaging, computed tomography (CT) scans, magnetic resonance imaging (MRI), etc. The manner by which images are generated during medical imaging depends on the particular technique.

For example, ultrasound imaging uses real time, non-invasive high frequency sound waves to produce ultrasound images, typically of organs, tissues, objects (e.g., fetus) inside the human body. Images produced or generated during medical imaging may be two-dimensional (2D), three-dimensional (3D), and/or four-dimensional (4D) images (essentially real-time/continuous 3D images). During medical imaging, imaging datasets (including, e.g., volumetric imaging datasets during 3D/4D imaging) are acquired and used in generating and rendering corresponding images (e.g., via a display) in real-time.

Use of medical imaging systems in conjunction with certain types of examination, however, poses certain challenges, particularly with respect to assessing outcome of the examination. For example, in some examinations determining presence of certain conditions or diseases by relying on detecting and/or identifying particular features in images generated and displayed in the course of the examinations, which may associated with and indicative of these conditions or diseases, may be difficult.

Further limitations and disadvantages of conventional and traditional approaches will become apparent to one of skill in the art, through comparison of such systems with some aspects of the present disclosure, as set forth in the remainder of the present application with reference to the drawings.

### BRIEF SUMMARY

System and methods are provided for robust intensity ranges for automatic lesion volume measurement, substantially as shown in and/or described in connection with at least one of the figures, as set forth more completely in the claims.

These and other advantages, aspects and novel features of the present disclosure, as well as details of one or more illustrated example embodiments thereof, will be more fully understood from the following description and drawings.

### BRIEF DESCRIPTION OF THE DRAWINGS

FIG. 1 is a block diagram illustrating an example medical imaging arrangement that may be configured for supporting robust intensity ranges for automatic lesion volume measurement.
FIG. 2 is a block diagram illustrating an example computed tomography (CT) system that may be configured for supporting robust intensity ranges for automatic lesion volume measurement.
FIG. 3 illustrates example medical imaging of lungs with standard reconstruction.
FIG. 4 illustrates example medical imaging of lungs with lung reconstruction.
FIG. 5 illustrates example medical imaging of lungs with lung reconstruction with data filtering.
FIG. 6 illustrates example medical imaging of lungs with quantification standard reconstruction.
FIG. 7 illustrates example medical imaging of lungs with quantification lung reconstruction.
FIG. 8 illustrates example medical imaging of lungs with quantification standard reconstruction with data filtering.
FIG. 9 illustrates example medical imaging of lungs with quantification lung reconstruction with data filtering.
FIG. 10 is a chart illustrating lesion extent attenuation based on different reconstruction kernels.
FIG. 11 is a chart illustrating impact of vessel subtraction on lesion detection.
FIG. 12 is a block diagram illustrating an example flowchart of pipeline based process for use of robust intensity ranges for automatic lesion volume measurement during medical imaging operations.

### DETAILED DESCRIPTION

Certain implementations in accordance with the present disclosure may be directed to robust intensity ranges for automatic lesion volume measurement. The foregoing summary, as well as the following detailed description of certain embodiments will be better understood when read in conjunction with the appended drawings. To the extent that the figures illustrate diagrams of the functional blocks of various embodiments, the functional blocks are not necessarily indicative of the division between hardware circuitry. Thus, for example, one or more of the functional blocks (e.g., processors or memories) may be implemented in a single piece of hardware (e.g., a general purpose signal processor or a block of random access memory, hard disk, or the like) or multiple pieces of hardware. Similarly, the programs may be stand-alone programs, may be incorporated as subroutines in an operating system, may be functions in an installed software package, and the like. It should be understood that the various embodiments are not limited to the arrangements and instrumentality shown in the drawings. It should also be understood that the embodiments may be combined, or that other embodiments may be utilized and that structural, logical and electrical changes may be made without departing from the scope of the various embodiments. The following detailed description is, therefore, not to be taken in a limiting sense, and the scope of the present disclosure is defined by the appended claims and their equivalents.

As used herein, an element or step recited in the singular and preceded with the word "a" or "an" should be understood as not excluding plural of said elements or steps, unless such exclusion is explicitly stated. Furthermore, references to "an exemplary embodiment," "various embodiments," "certain embodiments," "a representative embodiment," and the like are not intended to be interpreted as excluding the existence of additional embodiments that also incorporate the recited features. Moreover, unless explicitly stated to the contrary, embodiments "comprising," "including," or "having" an element or a plurality of elements having a particular property may include additional elements not having that property.

Also as used herein, the term "image" broadly refers to both viewable images and data representing a viewable image. However, many embodiments generate (or are configured to generate) at least one viewable image.

In addition, as used herein, the phrase "pixel" also includes embodiments where the data is represented by a "voxel." Thus, both the terms "pixel" and "voxel" may be used interchangeably throughout this document.

Furthermore, the term processor or processing unit, as used herein, refers to any type of processing unit that can carry out the required calculations needed for the various embodiments, such as single or multi-core: CPU, Accelerated Processing Unit (APU), Graphics Board, DSP, FPGA, ASIC, or a combination thereof.

It should be noted that various embodiments described herein that generate or form images may include processing for forming images that in some embodiments includes beamforming and in other embodiments does not include beamforming. For example, an image can be formed without beamforming, such as by multiplying the matrix of demodulated data by a matrix of coefficients so that the product is the image, and wherein the process does not form any "beams". In addition, forming of images may be performed using channel combinations that may originate from more than one transmit event (e.g., synthetic aperture techniques).

In various embodiments, processing to form images, including beamforming, is performed in software, firmware, hardware, or a combination thereof.

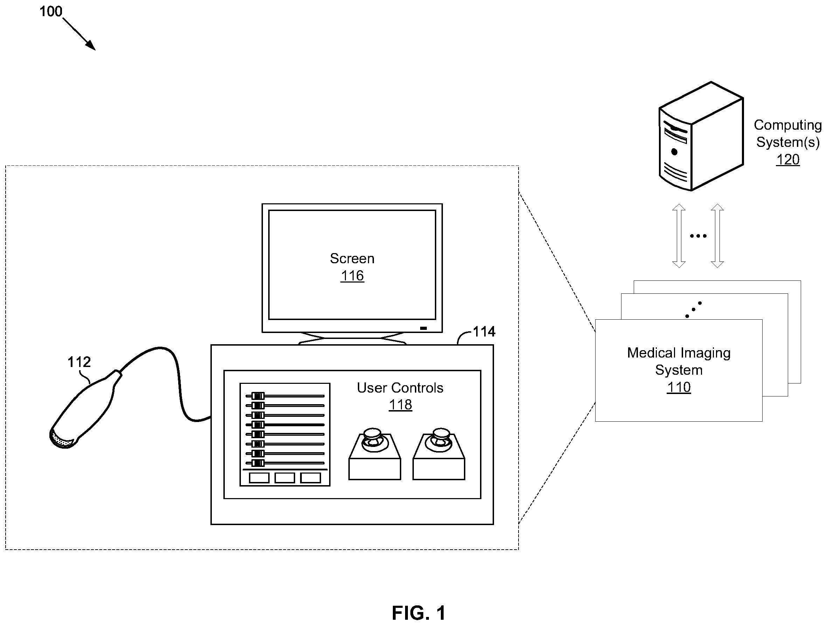

Fig. 1 is a block diagram illustrating an example medical imaging arrangement that may be configured for supporting robust intensity ranges for automatic lesion volume measurement. Shown in Fig. 1 is an example setup 100 that comprises one or more medical imaging systems 110 and one or more computing systems 120.

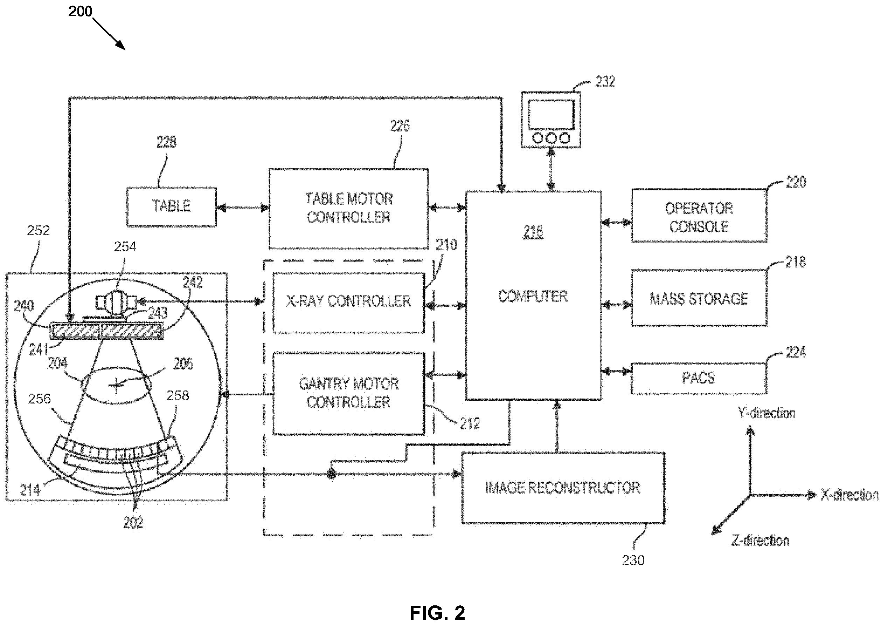

The medical imaging system 110 comprise suitable hardware, software, or a combination thereof, for supporting medical imaging-that is enabling obtaining data used in generating and/or rendering images during medical imaging exams. Examples of medical imaging include ultrasound imaging, computed tomography (CT) scans, magnetic resonance imaging (MRI), etc. This may entail capturing of particular type of data, in particular manner, which may in turn be used in generating data for the images. For example, the medical imaging system 110 may be a CT scan based system, configured for generating and/or rendering medical images based on CT scanning techniques. An example implementation of a CT system, which may correspond to the medical imaging system 110, is described in more detail with respect to Fig. 2.

As shown in Fig. 1, the medical imaging system 110 may comprise a scanner device 112, which may be portable and movable, and a display/control unit 114. The scanner device 112 may be configured for generating and/or capturing particular type of imaging signals (and/or data corresponding thereto), such as by being moved over a patient's body (or part thereof), and may comprise suitable circuitry for performing and/or supporting such functions. The scanner device 112 may be an ultrasound probe, MRI scanner, CT scanner, or any suitable imaging device.

The display/control unit 114 may be configured for displaying images (e.g., via a screen 116). In some instances, the display/control unit 114 may further be configured for generating the displayed images, at least partly. Further, the display/control unit 114 may also support user input/output. For example, the display/control unit 114 may provide (e.g., via the screen 116), in addition to the images, user feedback (e.g., information relating to the system, functions thereof, settings thereof, etc.). The display/control unit 114 may also support user input (e.g., via user controls 118), such as to allow controlling of the medical imaging. The user input may be directed to controlling display of images, selecting settings, specifying user preferences, requesting feedback, etc.

In some implementation, the medical imaging system 110 may also incorporate additional and dedicated computing resources, such as the one or more computing systems 120. In this regard, each computing system 120 may comprise suitable circuitry, interfaces, logic, and/or code for processing, storing, and/or communication data. The computing system 120 may be dedicated equipment configured particularly for use in conjunction with medical imaging, or it may be a general purpose computing system (e.g., personal computer, server, etc.) set up and/or configured to perform the operations described hereinafter with respect to the computing system 120. The computing system 120 may be configured to support operations of the medical imaging systems 110, as described below. In this regard, various functions and/or operations may be offloaded from the imaging systems. This may be done to streamline and/or centralize certain aspects of the processing, to reduce cost-e.g., by obviating the need to increase processing resources in the imaging systems.

The computing systems 120 may be set up and/or arranged for use in different ways. For example, in some implementations a single computing system 120 may be used; in other implementations multiple computing systems 120, either configured to work together (e.g., based on distributed-processing configuration), or separately, with each computing system 120 being configured to handle particular aspects and/or functions, and/or to process data only for particular medical imaging systems 110. Further, in some implementations, the computing systems 120 may be local (e.g., co-located with one or more medical imaging systems 110, such within the same facility and/or same local network); in other implementations, the computing systems 120 may be remote and thus can only be accessed via remote connections (e.g., via the Internet or other available remote access techniques). In a particular implementation, the computing systems 120 may be configured in cloud-based manner, and may be accessed and/or used in substantially similar way that other cloud-based systems are accessed and used.

Once data is generated and/or configured in the computing system 120, the data may be copied and/or loaded into the medical imaging systems 110. This may be done in different ways. For example, the data may be loaded via directed connections or links between the medical imaging systems 110 and the computing system 120. In this regard, communications between the different elements in the setup 100 may be done using available wired and/or wireless connections, and/or in accordance any suitable communication (and/or networking) standards or protocols. Alternatively, or additionally, the data may be loaded into the medical imaging systems 110 indirectly. For example, the data may be stored into suitable machine readable media (e.g., flash card, etc.), which are then used to load the data into the medical imaging systems 110 (on-site, such as by users of the systems (e.g., imaging clinicians) or authorized personnel), or the data may be downloaded into local communication-capable electronic devices (e.g., laptops, etc.), which are then used on-site (e.g., by users of the systems or authorized personnel) to upload the data into the medical imaging systems 110, via direct connections (e.g., USB connector, etc.).

In operation, the medical imaging system 110 may be used in generating and presenting (e.g., rendering or displaying) images during medical exams, and/or in supporting user input/output in conjunction therewith. The images may be 2D, 3D, and/or 4D images. The particular operations or functions performed in the medical imaging system 110 to facilitate the generating and/or presenting of images depends on the type of system-that is, the manner by which the data corresponding to the images is obtained and/or generated.

In various implementations in accordance with the present disclosure, medical imaging systems (e.g., the medical imaging system 110) may be configured to support use of robust intensity ranges for automatic lesion volume measurement. In this regard, medical imaging operations may pose various challenges, particularly due to the complexities, difficulties and tediousness associated with various aspects of these operations. For example, medical imaging entails and/or requires careful analysis of the images generated using medical imaging techniques (e.g., CT scans), such as to detect particular medical conditions. In this regard, review and analysis of medical images may involve examining particular organs (e.g., lungs, heart, liver, vessels etc.) to identify particular features therein or characteristics thereof, which may be associated with and indicative of the presence of such medical conditions.

The review and analysis of medical images is, however, a tedious task, and thus at least some of these features may be missed or not adequately identified or accounted for, which may result in misdiagnosis, inaccurate measurements, or other errors. Accordingly, medical imaging operations may be improved by incorporating measures for enhancing detection of certain features in medical images, specifically where such features may be indicative of particular medical conditions. For example, medical imaging based examination of the lungs, particularly in conjunction with certain respiratory diseases, such as the coronavirus disease 2019 (COVID-19), may be improved by enhancing the ability to reliably and accurately identify pertinent features associated with such diseases. In such use case scenarios, bilateral distribution of lesions may be an important biomarker, and as such medical imaging based examination of the lungs (e.g., using CT scan of the lungs) may be improved by incorporating measures for ensuring and/or enhancing reliable and accurate detection of legions that may be indicative of such respiratory diseases (e.g., COVID-19).

Accordingly, in various example implementations in accordance with the present disclosure, medical imaging systems may be configured to incorporate use of automatic computation of parameters and/or settings used in lesion detection. This may be done by incorporating measures for automatically computing the lesion burden of a segmented organ (e.g., lungs) in a robust and reproducible way.

In an example implementation and use scenario(s) based thereon in the domain of CT lung imaging/review, where the user may typically manually set or adjust particular parameters or settings that are pertinent to lesion detection, such as HU (Hounsfield scale (HU)) thresholds, which may be used in measuring the total volume of lesions inside the lungs. The manual setting or adjusting of such values, however, may be error prone and may lead to inconsistent measurements if the acquisition settings are not properly taken into account, which may result in misdiagnosis.

In accordance with the present disclosure, parameters or settings used or pertinent to lesion detection may be automatically set or adjusted, to optimize lesion detection. For example, this may be done by use of automatic processing pipeline, where an input image is first pre-processed, and some HU ranges are automatically determined to measure the pertinent pathological region(s) in a more consistent manner that may be less influenced by acquisition settings such as reconstruction parameters (reconstruction kernel, slice spacing, pixel size, etc.), contrast injection or respiratory phase, and the like. In this regard, as noted, HU thresholds and settings thereof may be relevant, particularly for lesions detection and segmentation, and as such may be implemented and used in, e.g., COVID related use case scenarios. Thus, tabulated HU ranges may be used, leading to good results even for consolidation/ground glass distinction.

Although these solutions may have the advantage of being easily adjustable and explainable (unlike, e.g., in artificial intelligence (AI) based approaches), such solutions may still have some shortcomings-e.g., may still need some manual adjustments, which may be tedious and/or challenging, especially under various clinical conditions. For instance, sharp reconstruction kernels may lead to more noise in the image(s), which may have an impact on attenuation area measured in the image using fixed intensity thresholds. There are also other factors of variability, such as slice thickness, image resolution, contrast injection, respiratory phase, etc. Implementations in accordance with the present disclosure may solve these problems and/or overcome these and other challenges present in any existing solutions, such as by building processed image(s) along with automatic HU ranges so that the measurements are more reproducible on different types of acquisitions and patient conditions.

In an example use scenario using an example implementation in accordance with the present disclosure, generated image may be preprocessed to reduce the impact of the reconstruction technique (e.g., noise reduction, resampling). Robust and reproducible intensity ranges (e.g., HU thresholds) are used to measure attenuation area(s) within a segmented organ (e.g., lungs). The intensity ranges are adjusted automatically to compensate for the variability induced by the acquisition technique and/or patient conditions. The intensity ranges may be determined by finding the optimal thresholds which may best approximate an AI computed lesion map. This may be done using pre-generated and/or pre-trained AI models. Examples of use of automatic setting and adjustment of lesion related parameters in conjunction with medical imaging examination of the lungs are shown and described in more detail below.

In some implementations, processing resources (e.g., with the imaging systems, the computing systems, etc.) may be configured to implement and/or use artificial intelligence and/or machine learning techniques to support and/or facilitate the generating and training of AI models. For example, suitable processing resources (e.g., processors and the like) may be configured to implement and/or use deep learning techniques and/or algorithms, such as by use of deep neural networks (e.g., a convolutional neural network (CNN)), and/or may utilize any suitable form of artificial intelligence image analysis techniques or machine learning processing functionality, which may be configured to analyze acquired medical images, such as to identify, segment, label, and track structures (or tissues thereof) meeting particular criteria and/or having particular characteristics.

In an example implementation, a deep neural network may be implemented, comprising, e.g., an input layer, an output layer, and one or more hidden layers in between the input and output layers. Each of the layers may be made up of a plurality of processing nodes that may be referred to as neurons. For example, the deep neural network may include an input layer having a neuron for each pixel or a group of pixels from a scan plane of an anatomical structure, and the output layer may have a neuron corresponding to a plurality of pre-defined structures or types of structures (or tissue(s) therein). Each neuron of each layer may perform a processing function and pass the processed medical image information to one of a plurality of neurons of a downstream layer for further processing.

The neurons of the deep neural network(s) may be trained (e.g., using medical images or datasets corresponding thereto), such as to identify particular structures and/or tissues (or types thereof), such as using databases(s) of classified medical images of various structures. For example, medical images of particular organs, structures, etc. may be used in the training, with respect to the characteristics of the particular structure(s), such as the appearance of structure edges, the appearance of structure shapes based on the edges, the positions of the shapes relative to landmarks in the medical image data, and the like, and/or with respect to characteristics of particular tissues (e.g., softness thereof). In various implementations, databases of training images may be maintained in suitable data storage medium and used in training functions.

In some implementations the functions or processes used in conjunction with use of robust intensity ranges for automatic lesion volume measurement may be done locally (directly within the medical imaging system or a local system) or remotely (in the Cloud, remote data center, etc.). This may allow for distributed operation-e.g., with images being generated in one location, at least some of the processing relating to the automatic lesion volume measurement being done in another location, and the displaying and examination of the images being done in yet another location.

FIG. 2 is a block diagram illustrating an example computed tomography (CT) system that may be configured for supporting robust intensity ranges for automatic lesion volume measurement. Shown in Fig. 2 is a computed tomography (CT) system 200.

The CT system 200 may correspond to the CT based implementation of the medical imaging system 110 of Fig. 1. In this regard, the CT system 200 may be configured for providing computed tomography (CT) based imaging and related functions, and as such may comprise suitable circuitry, interfaces, logic, and/or code for performing and/or supporting CT imaging and/or these related function.

For example, as illustrated in FIG. 2, the CT system 200 may be used to conduct CT imaging on a subject 204 (e.g., placed on a movable table 228), such as a patient, an inanimate object, one or more manufactured parts, and/or foreign objects such as dental implants, stents, and/or contrast agents present within patient's body. The CT system 200 may comprise a gantry 252, which in turn, may further comprise at least one x-ray radiation source 254 configured to project a beam of x-ray radiation 256 for use in imaging the subject 204. The x-ray radiation source 254 may comprise an x-ray tube and a target.

The x-ray tube generate x-rays by accelerating and focusing a high-energy beam of electrons onto a rotating target. As individual electrons strike the target, the energy released by interacting with the atoms of the target produces x-ray photons isotropically under a polychromatic spectrum, a maximum energy of the x-ray photons matching that of the incident electrons. The x-ray photons leave the tube through a window that defines an x-ray beam. The beam can then be collimated and conditioned using collimator blades and filter(s).

Specifically, the radiation source 254 may be configured to project the x-rays 256 towards a detector array 258 positioned on the opposite side of the gantry 252. Although FIG. 2 depicts only a single radiation source 254, in certain embodiments, multiple radiation sources may be employed to project a plurality of x-rays 256 for acquiring projection data corresponding to the subject 204 at different energy levels. The radiation source may comprise an x-ray target manufactured of graphite and metal. In certain embodiments, the CT system 200 may further comprise image processing components (e.g., computing device 216, image reconstructor 230, etc.) configured to reconstruct images of a target volume of the subject 204 using an iterative or analytic image reconstruction method. For example, the image processing components may use an analytic image reconstruction approach such as Filtered BackProjection (FBP) to reconstruct images of a target volume of the subject 204. As another example, the image processing components may use an iterative image reconstruction approach such as advanced statistical iterative reconstruction (ASIR), conjugate gradient (CG), maximum likelihood expectation maximization (MLEM), model-based iterative reconstruction (MBIR), and so on to reconstruct images of a target volume of the subject 204.

The detector array 258 may further comprise a plurality of detector elements 202 that together sense the x-ray beam 256 (see FIG. 2) that pass through the subject 204 to acquire corresponding projection data. In an example embodiment, the detector array 258 may be fabricated in a multi-slice configuration comprising the plurality of rows of cells or detector elements 202. In such a configuration, one or more additional rows of the detector elements 202 are arranged in a parallel configuration for acquiring the projection data.

A filter carriage 240 may be mounted within gantry 252 between radiator source 254 and the subject 204. The carriage 240 may travel in and out of the beam in the z-direction while the beam is substantially in the y-direction. Two different bowtie filters, a first bowtie filter 241 and a second bowtie filter 242 are shown in FIG. 2 as an example. The first bowtie filter 241 is housed within a first slot formed in a cavity of the carriage and wherein the second bowtie filter 242 is housed within a second slot formed in the cavity of the carriage, the first slot separated from the second slot via a tab. The bowtie filters are shown here in rectangular shape as an example.

Each and every bowtie filter is rigid and non-deformable. The bowtie filters may alternatively have different shapes and material constructions to provide proper x-ray special spectrum for imaging various types of anatomies. A hardening filter 243 is shown coupled to the carriage 240 between the two bowtie filters 241 and 242. The hardening filter 243 may at least partially overlap with each of the first bowtie filter 241 and the second bowtie filter 242. In one example, the hardening filter only partially overlaps with each of the first bowtie filter 241 and the second bowtie filter 242. In another example, the hardening filter 243 may completely overlap with one of the first bowtie filter 241 and the second bowtie filter 242. The hardening filter 243 may comprise each of a rectangular support structure, and one or more rectangular metallic sheets stacked under the support structure. The rectangular support structure may be made of aluminum and the one or more rectangular metallic sheets may be made of copper with each of the one or more rectangular metallic sheets having a different thickness.

In an example use scenario, the x-ray beam 256 passes through the hardening filter 243 and the second bowtie filter 242. However, the carriage 240 may be moved to a position such that the beam may pass through a bowtie filter (first or second) and not through the hardening filter. As an example, if the carriage 240 is moved further towards the left, the beam may solely pass through the second bowtie filter 243. In this way, it is possible to pass the beam through a bowtie filter and each of a hardening filter and a bowtie filter.

The bowtie filter may change the spatial distribution of the radiation beam in the axial plane of the imaging subject (such as a patient). For example, the re-distributed radiation beam may have higher energy at the center and lower energy at the periphery of the subject. Each of the bowtie filters may be designed to image a specific anatomy or section of the human body, such as head, chest, and abdomen. During imaging, one of the bowtie filters may be selected based on the anatomy of the subject to be scanned, and the selected filter may be placed into the radiation beam path. Responsive to a change in the anatomy, the filter may be changed from one to another. Based on a nature of the scan, the carriage may be positioned such that the hardening filter may or may not be placed in the radiation beam path. The hardening filter may attenuate the beam and remove low energy components thereby conditioning the beam for specific scans such as a scout scan.

A filter driving system (not shown) may be coupled to the carriage 240 to move the one or more filters into and out of the radiation beam path. In one embodiment, a motor may couple the filters in the carriage through a shaft. The bowtie filters may be switched from one to another and/or a hardening filter may be introduced or removed from the beam path by translating the filters along the shaft by rotating the shaft with a motor. One of the filters may be selected and translated into the x-ray beam between the radiation source and the imaging subject to image a specific section of the human body. Computing device 216 may send command to the motor of the filter driving system to move the selected filter in to the radiation beam. The filter driving system may also send filter position information back to the computing device 216.

In certain embodiments, the CT system 200 may be configured to traverse different angular positions around the subject 204 for acquiring desired projection data. Accordingly, the gantry 252 and the components mounted thereon (such as the radiation source 254, the filter housing 240, and the detector 202) may be configured to rotate about a center of rotation 206 for acquiring the projection data, for example, at different energy levels. Alternatively, in embodiments where a projection angle relative to the subject 204 varies as a function of time, the mounted components may be configured to move along a general curve rather than along a segment of a circle.

In one embodiment, the CT system 200 may comprise a control mechanism 208 to control movement of the components such as rotation of the gantry 252 and the operation of the x-ray radiation source 254. In certain embodiments, the control mechanism 208 may further comprise an x-ray controller 210 configured to provide power and timing signals to the radiation source 254. Additionally, the control mechanism 208 may comprise a gantry motor controller 212 configured to control a rotational speed and/or position of the gantry 252 based on imaging requirements.

In certain embodiments, the control mechanism 208 may further comprise a data acquisition system (DAS) 214 configured to sample analog data received from the detector elements 202 and convert the analog data to digital signals for subsequent processing. The data sampled and digitized by the DAS 214 is transmitted to a computing device (also referred to as processor) 216. In one example, the computing device 216 stores the data in a storage device 218. The storage device 218, for example, may comprise a hard disk drive, a floppy disk drive, a compact disk-read/write (CD-R/W) drive, a Digital Versatile Disc (DVD) drive, a flash drive, and/or a solid-state storage device.

Additionally, the computing device 216 provides commands and parameters to one or more of the DAS 214, the x-ray controller 210, and the gantry motor controller 212 for controlling system operations such as data acquisition and/or processing. In certain embodiments, the computing device 216 controls system operations based on operator input. The computing device 216 receives the operator input, for example, comprising commands and/or scanning parameters via an operator console 220 operatively coupled to the computing device 216. The operator console 220 may comprise a keyboard or a touchscreen to allow the operator to specify the commands and/or scanning parameters.

Although FIG. 2 illustrates only one operator console 220, more than one operator console may be coupled to the CT system 200, for example, for inputting or outputting system parameters, requesting examinations, and/or viewing images. Further, in certain embodiments, the CT system 200 may be coupled to multiple displays, printers, workstations, and/or similar devices located either locally or remotely, for example, within an institution or hospital, or in an entirely different location via one or more configurable wired and/or wireless networks such as the Internet and/or virtual private networks.

In one embodiment, for example, the CT system 200 either may comprise, or is coupled to a picture archiving and communications system (PACS) 224. In an exemplary implementation, the PACS 224 is further coupled to a remote system such as a radiology department information system, hospital information system, and/or to an internal or external network (not shown) to allow operators at different locations to supply commands and parameters and/or gain access to the image data.

The computing device 216 uses the operator-supplied and/or system-defined commands and parameters to operate a table motor controller 226, which in turn, may control a motorized table 228. Particularly, the table motor controller 226 moves the table 228 for appropriately positioning the subject 204 in the gantry 252 for acquiring projection data corresponding to the target volume of the subject 204.

The DAS 214 samples and digitizes the projection data acquired by the detector elements 202. Subsequently, an image reconstructor 230 uses the sampled and digitized x-ray data to perform high-speed reconstruction. Although FIG. 2 illustrates the image reconstructor 230 as a separate entity, in certain embodiments, the image reconstructor 230 may form part of the computing device 216. Alternatively, the image reconstructor 230 may be absent from the CT system 200 and instead the computing device 216 may perform one or more functions of the image reconstructor 230. Moreover, the image reconstructor 230 may be located locally or remotely, and the image reconstructor 230 may be operatively connected to the system 250 using a wired or wireless network. Particularly, one exemplary embodiment may use computing resources in a "cloud" network cluster for the image reconstructor 230.

In one embodiment, the image reconstructor 230 stores the images reconstructed in the storage device 218. Alternatively, the image reconstructor 230 transmits the reconstructed images to the computing device 216 for generating useful patient information for diagnosis and evaluation. In certain embodiments, the computing device 216 transmits the reconstructed images and/or the patient information to a display 232 communicatively coupled to the computing device 216 and/or the image reconstructor 230.

In accordance with the present disclosure, the CT system 200 may be configured to incorporating and/or supporting use of automatic computation and setting of parameters and/or settings used in lesion detection, particularly by use of robust intensity ranges for automatic lesion volume measurement, as described with respect to FIG. 1. This may be done, for example, by incorporating into CT system 200 measures for automatically setting parameters and settings applied during CT imaging based examinations, such as examination of the lungs when detecting certain conditions or diseases (e.g., COVID-19).

For example, in instances where the CT system 200 is utilized in conjunction with CT imaging based lung examinations, parameters or settings that are pertinent to lesion detection, such as HU (Hounsfield scale (HU)) thresholds may be set or adjusted automatically, to optimize lesion detection. This may be done by, for example, incorporating into CT system 200 use of automatic pipeline, where the input image is first pre-processed, and some HU ranges are automatically determined to optimally measure the pathological region(s) associated with the lesions that may be biomarkers of the diseases. The ranges may be pre-determined and pre-stored into the CT system 200 and used thereafter. For example, this may be done by use of artificial intelligence (AI) models, as described herein. Examples of use of automatic setting and adjustment of lesion related parameters in conjunction with medical imaging examination of the lungs are shown and described in more detail below.

Accordingly, in an example use case scenario in accordance with the present disclosure, pre-defined model (e.g., an AI model, such as a deep learning model) are used to segment different regions of the lungs during CT based examination of the lungs, with the segmenting directed to such structures and/or features as bronchi, pulmonary vessels, lesions (GGO, crazy paving, consolidation), pleural effusion, etc. The model is trained to be robust to acquisition technique and patient's state, and is configured to provide automatic HU ranges that best approximates segmented lesion area(s), combined with non-parenchyma structure removal (e.g., vessels, bronchi, pleural effusion). Thus, the resulting method may be HU based, and is explainable and adjustable. Such approach may yield better reproducibility in the measure of the lesion extent for a given patient (e.g., follow-up), or across patients (comparative study).

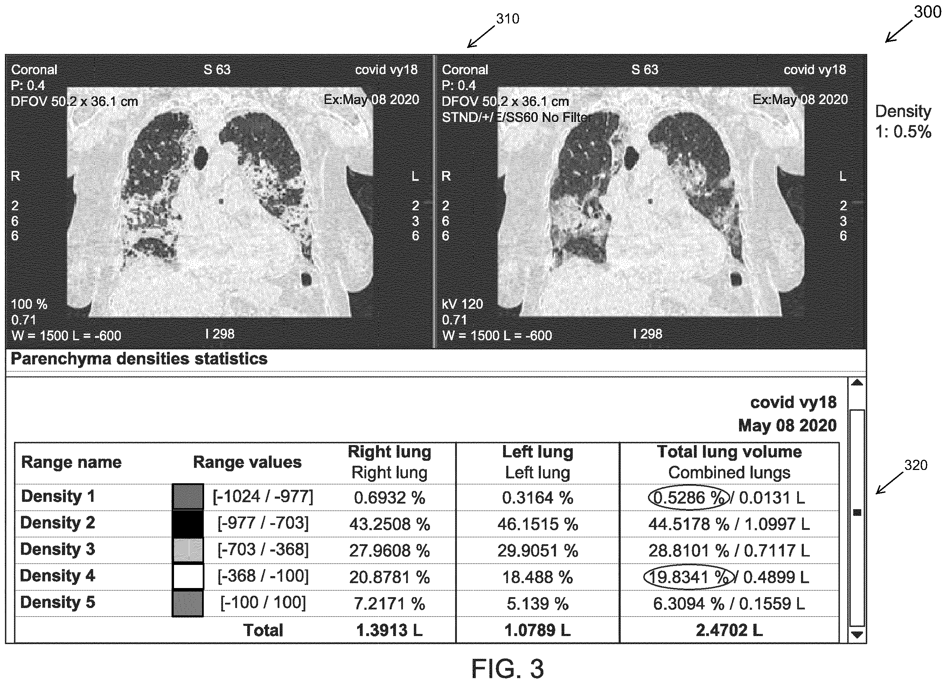

FIG. 3 illustrates example medical imaging of lungs with standard reconstruction. Shown in FIG. 3 is screenshot of interface 300 utilized during an example CT examination of a patient (particularly of the chest area).

The interface 300 includes a subsection where images 310 are displayed, with these images generated based on standard reconstruction of CT acquisition during the CT examination. In this regard, as shown in FIG. 3 the images 310 may be for a patient with respiratory condition-namely, COVID-19. The interface 300 also includes subsection 320 with information relating to the images 310, particularly density related information.

As shown in FIG. 3, the images 310 may comprise regions (e.g., displayed using different colors in the images) corresponding to a plurality of densities (e.g., density 1 through density 5). The regions may be identified (and thus displayed accordingly-e.g., using a color assigned to the corresponding density), based on processing of the image data using a plurality of range values (e.g., intensity (HU) thresholds) which may be used to define and detect these different densities-that is, with range of values (or any other settings, though not shown) for each the densities (density 1 through density 5). In existing solutions, the user sets the range values (and/or other presets) manually when viewing the images. In accordance with the present disclosure, however, at least some of the range values may be automatically determined in the system, particularly using pre-defined models (e.g., AI models) as described herein.

As illustrated in FIG. 3, along with the image, the range values for the different densities may also be displayed in subsection 320, along with the corresponding results for the densities-e.g., showing for each density the corresponding volume percentage for each individual lung (left lung and right lung), as well as aggregate/total volume (e.g., in liters) and volume percentage for both lungs.

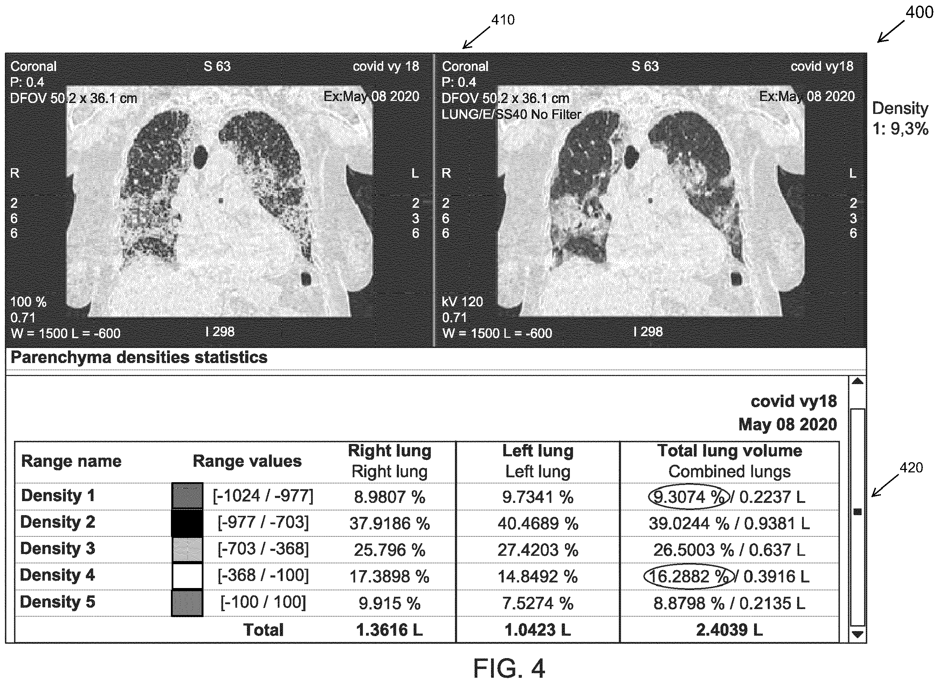

FIG. 4 illustrates example medical imaging of lungs with lung reconstruction. Shown in FIG. 4 is screenshot of interface 400 utilized during an example CT examination of a patient (particularly of the chest area). The interface 400 includes a subsection where images 410 are displayed, with these images generated during the CT examination.

In this regard, as shown in FIG. 4 the images 410 are generated using lung reconstruction of the same CT acquisition used to generate the images 310 of FIG. 3 using standard reconstruction. The interface 400 similarly includes subsection 420 with information relating to the images 410, particularly density related information.

As shown in FIG. 4, the images 410 may similarly comprise regions corresponding to a plurality of densities (e.g., density 1 through density 5), which may be generated and/or identified using the same plurality of range values (and/or other settings) used in generating images 310 of FIG. 3. However, the use of lung reconstruction allows for enhanced representation of regions or areas corresponding to certain densities (e.g., density 1) which may be associated with certain respiratory conditions (e.g., COVID-19).

The use of lung reconstruction may result enhancement in lesion detection, evidenced in increase in region corresponding to density 1, as illustrated in FIG. 4 (both within the images 410 and the information provided in subsection 420).

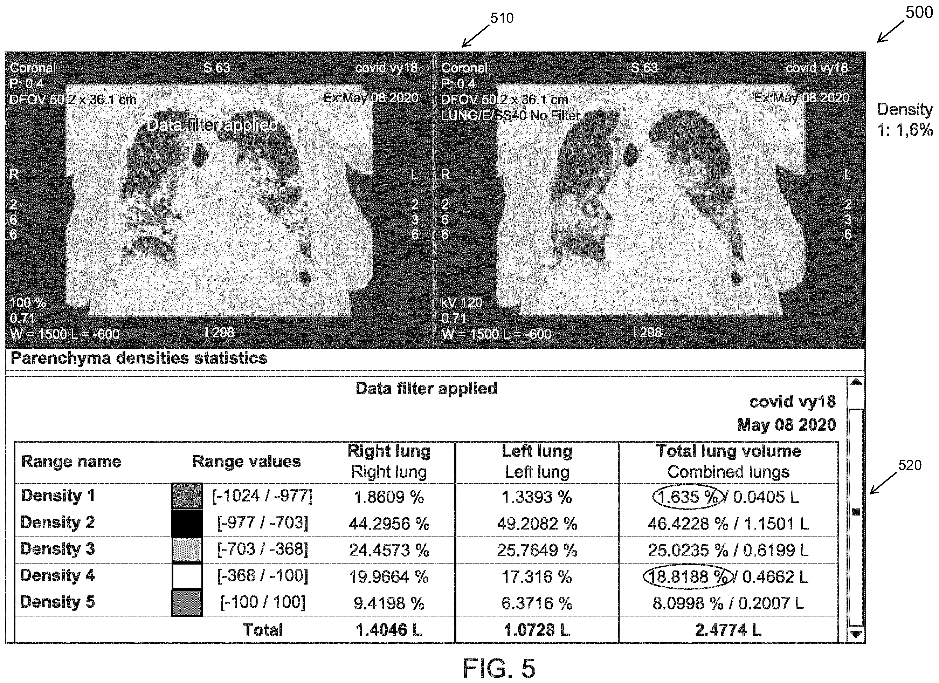

FIG. 5 illustrates example medical imaging of lungs with lung reconstruction with data filtering. Shown in FIG. 5 is screenshot of interface 500 utilized during an example CT examination of a patient (particularly of the chest area). The interface 500 includes a subsection where images 510 are displayed, with these images generated during the CT examination.

In this regard, as shown in FIG. 5 the images 510 are generated using lung reconstruction, and additionally with data filtering, of the same CT acquisition used to generate, using standard reconstruction, the images 310 of FIG. 3. The interface 500 similarly includes subsection 520 with information relating to the images 510, particularly density related information.

As shown in FIG. 5, the images 510 may similarly comprise regions corresponding to a plurality of densities (e.g., density 1 through density 5), which may be generated and/or identified using the same plurality of range values (and/or other settings) used in generating images 310 of FIG. 3. However, the use of lung reconstruction and application of data filtering (e.g., low-pass filtering for reducing blurring by decreasing noise in the images) further enhanced accuracy of identification of regions or areas corresponding to certain densities (e.g., density 1) which may be associated with certain respiratory conditions (e.g., COVID-19).

The use of lung reconstruction with data filtering may result in further enhancement in lesion detection, particularly more accurate detection of such lesion. This is illustrated in FIG. 5 (both within the images 510 and the information provided in subsection 520), as the region corresponding to density 1 is increased compared to images 310, but reduced compared to images 410 as subsections that may have been inaccurately (e.g., due to noise) identified as corresponding to density 1 are excluded.

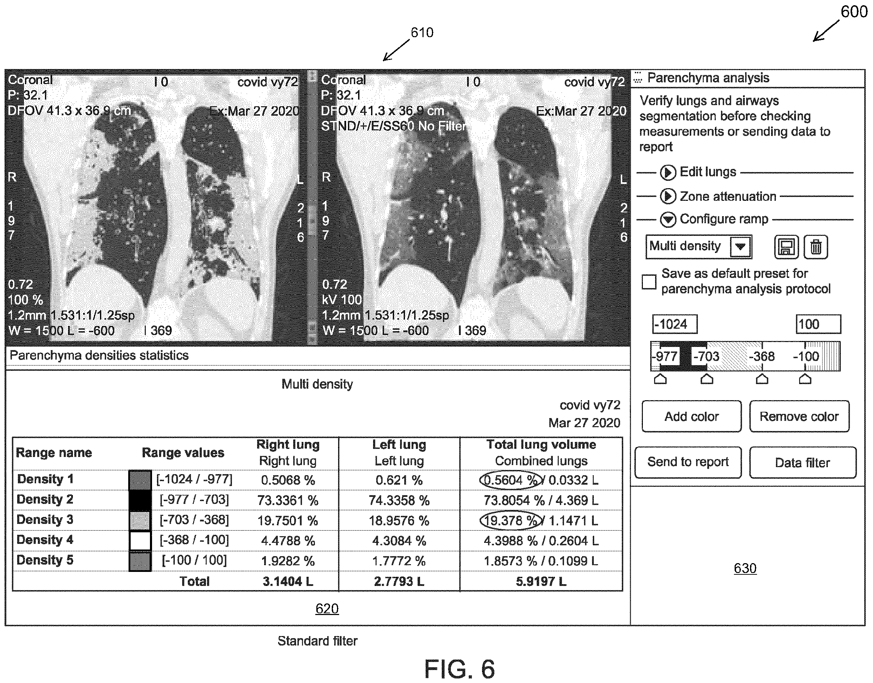

FIG. 6 illustrates example medical imaging of lungs with quantification standard reconstruction. Shown in FIG. 6 is screenshot of interface 600 utilized during an example CT examination of a patient (particularly of the chest area). The interface 600 includes a subsection where images 610 are displayed, with these images generated based on quantification standard reconstruction of CT acquisition during the CT examination. In this regard, as shown in FIG. 6 the images 610 may be for a patient with respiratory condition-namely, COVID-19.

The interface 600 also includes subsection 620 with information relating to the images 610, particularly density related information. Further, the interface 600 includes a subsection 630 to allow the user to provide various inputs relating to the CT examination, including setting presets or parameters relating to the lesion detection. For example, subsection 630 may comprise input means (dials, buttons, etc.) for setting range values used in detecting different regions in the images based on density.

As shown in FIG. 6, the images 610 may comprise regions (e.g., displayed using different colors in the images) corresponding to a plurality of densities (e.g., density 1 through density 5). The regions may be identified (and thus displayed accordingly-e.g., using a color assigned to the corresponding density), based on processing of the image data using a plurality of range values (e.g., intensity (HU) thresholds) which may be used to define and detect these different densities-that is, with range of values (or any other settings, though not shown) for each the densities (density 1 through density 5). In existing solutions, the user sets the range values (and/or other presets) manually when viewing the images. In accordance with the present disclosure, however, at least some of the range values may be automatically determined in the system, particularly using pre-defined models (e.g., AI models) as described herein.

As illustrated in FIG. 6, along with the image, the range values for the different densities may also be displayed in subsection 620, along with the corresponding results for the densities-e.g., showing for each density the corresponding volume percentage for each individual lung (left lung and right lung), as well as aggregate/total volume (e.g., in liters) and volume percentage for both lungs. In some implementations, once the values are determined automatically, the user may adjust some of the values (e.g., based on displayed imaged, to enhance quality). For example, the subsection 630 may comprise a portion for controlling the plurality of range values, with input means (e.g., dials, buttons, etc.) to modify some of the values. As shown in FIG. 6, the user may be able to adjust (e.g., by sliding) the values corresponding to the automatically determined thresholds.

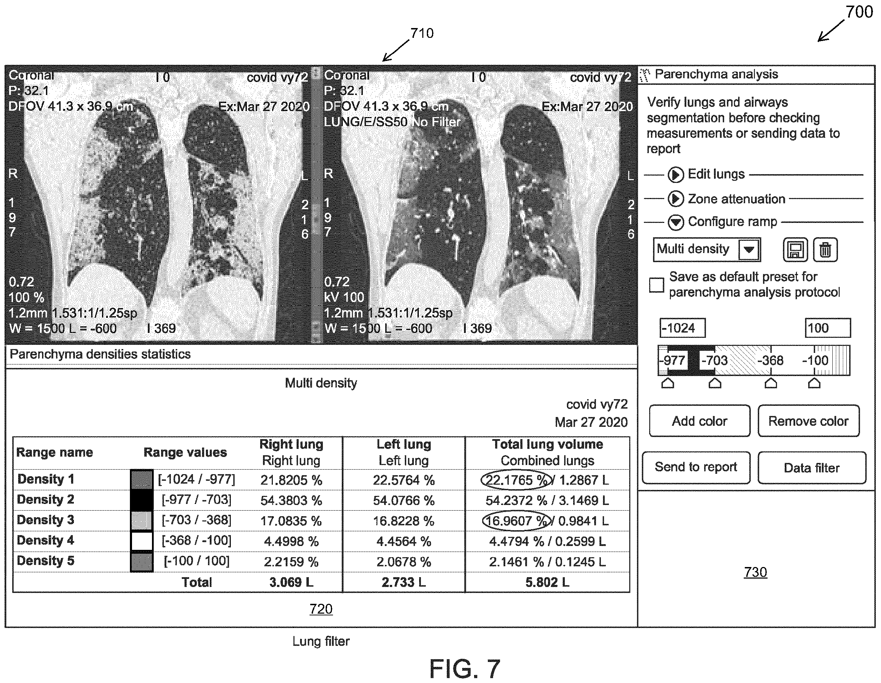

FIG. 7 illustrates example medical imaging of lungs with quantification lung reconstruction. Shown in FIG. 7 is screenshot of interface 700 utilized during an example CT examination of a patient (particularly of the chest area). The interface 700 includes a subsection where images 710 are displayed, with these images generated during the CT examination.

In this regard, as shown in FIG. 7 the images 710 are generated using quantification lung reconstruction of the same CT acquisition used to generate the images 610 of FIG. 6 using quantification standard reconstruction. The interface 700 similarly includes subsection 720 with information relating to the images 710, particularly density related information. Further, the interface 700 includes a subsection 730 to allow the user to provide various inputs relating to the CT examination, including setting presets or parameters relating to the lesion detection. In this regard, the subsection 730 may comprise means facilitating adjusting automatically-determined values substantially as described with respect to subsection 630 in interface 600.

As shown in FIG. 7, the images 710 may similarly comprise regions corresponding to a plurality of densities (e.g., density 1 through density 5), which may be generated and/or identified using the same plurality of range values (and/or other settings) used in generating images 610 of FIG. 6. However, the use of quantification lung reconstruction allows for enhanced representation of regions or areas corresponding to certain densities (e.g., density 1) which may be associated with certain respiratory conditions (e.g., COVID-19).

The use of quantification lung reconstruction may result enhancement in lesion detection, evidenced in increase in region corresponding to density 1, as illustrated in FIG. 7 (both within the images 710 and the information provided in subsection 720).

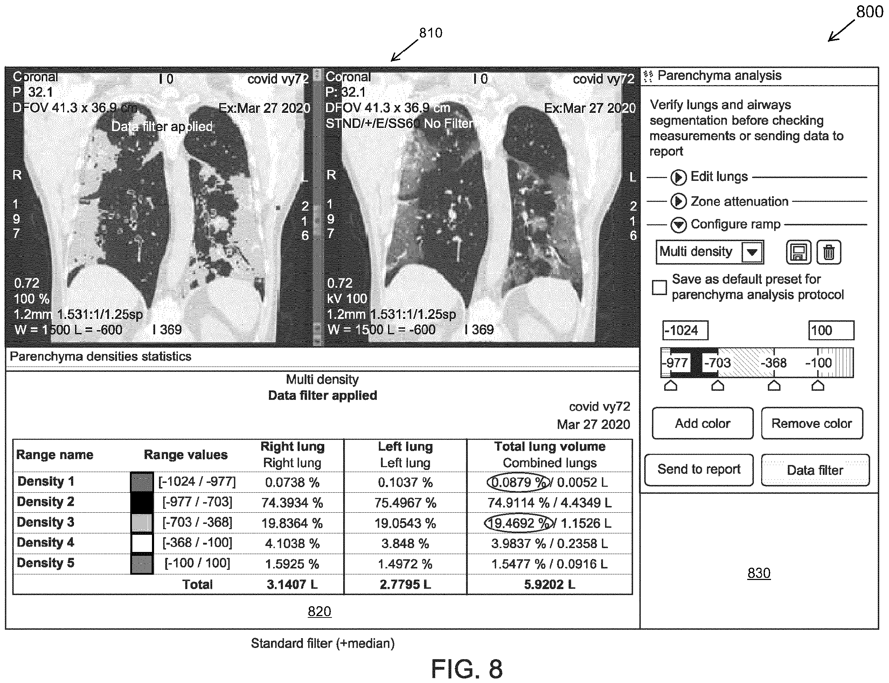

FIG. 8 illustrates example medical imaging of lungs with quantification standard reconstruction with data filtering. Shown in FIG. 8 is screenshot of interface 800 utilized during an example CT examination of a patient (particularly of the chest area). The interface 800 includes a subsection where images 810 are displayed, with these images generated based on quantification standard reconstruction, with data filtering (e.g., low-pass filtering for reducing blurring), of CT acquisition during the CT examination. In this regard, as shown in FIG. 8 the images 810 may be for a patient with respiratory condition-namely, COVID-19.

The interface 800 also includes subsection 820 with information relating to the images 810, particularly density related information. Further, the interface 800 includes a subsection 830 to allow the user to provide various inputs relating to the CT examination, including setting presets or parameters relating to the lesion detection. For example, subsection 830 may comprise input means (dials, buttons, etc.) for setting range values used in detecting different regions in the images based on density. In this regard, the subsection 830 may comprise means facilitating adjusting automatically-determined values substantially as described with respect to subsection 630 in interface 600.

As shown in FIG. 8, the images 810 may comprise regions (e.g., displayed using different colors in the images) corresponding to a plurality of densities (e.g., density 1 through density 5). The regions may be identified (and thus displayed accordingly-e.g., using a color assigned to the corresponding density), based on processing of the image data using a plurality of range values (e.g., intensity (HU) thresholds) which may be used to define and detect these different densities-that is, with range of values (or any other settings, though not shown) for each the densities (density 1 through density 5). In existing solutions, the user sets the range values (and/or other presets) manually when viewing the images. In accordance with the present disclosure, however, at least some of the range values may be automatically determined in the system, particularly using pre-defined models (e.g., AI models) as described herein.

As illustrated in FIG. 8, along with the image, the range values for the different densities may also be displayed in subsection 820, along with the corresponding results for the densities-e.g., showing for each density the corresponding volume percentage for each individual lung (left lung and right lung), as well as aggregate/total volume (e.g., in liters) and volume percentage for both lungs.

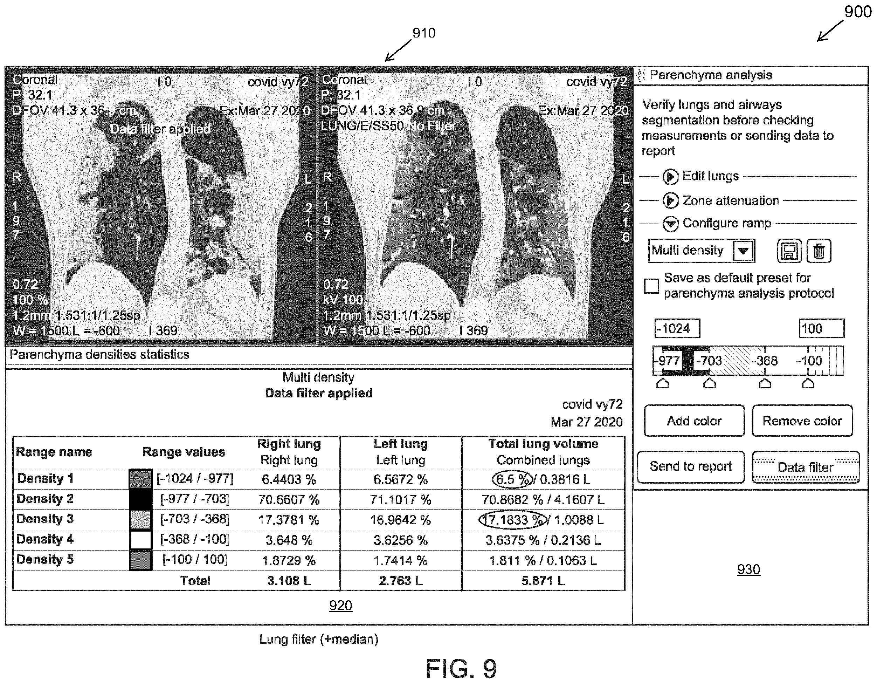

FIG. 9 illustrates example medical imaging of lungs with quantification lung reconstruction with data filtering. Shown in FIG. 9 is screenshot of interface 900 utilized during an example CT examination of a patient (particularly of the chest area). The interface 900 includes a subsection where images 910 are displayed, with these images generated during the CT examination.

In this regard, as shown in FIG. 9 the images 910 are generated using quantification lung reconstruction with data filtering, of the same CT acquisition used to generate the images 810 of FIG. 8 using quantification standard reconstruction with data filtering. The interface 900 similarly includes subsection 920 with information relating to the images 910, particularly density related information, and a subsection 930 to allow the user to provide various inputs relating to the CT examination, including setting presets or parameters relating to the lesion detection. In this regard, the subsection 930 may comprise means facilitating adjusting automatically-determined values substantially as described with respect to subsection 630 in interface 600.

As shown in FIG. 9, the images 910 may similarly comprise regions corresponding to a plurality of densities (e.g., density 1 through density 5), which may be generated and/or identified using the same plurality of range values (and/or other settings) used in generating images 810 of FIG. 8. However, the use of quantification lung reconstruction further enhanced accuracy of identification of regions or areas corresponding to certain densities (e.g., density 1) which may be associated with certain respiratory conditions (e.g., COVID-19), even with data filtering applied in both cases. This is illustrated in FIG. 9 (both within the images 910 and the information provided in subsection 920), as the region corresponding to density 1 is detected more accurately.

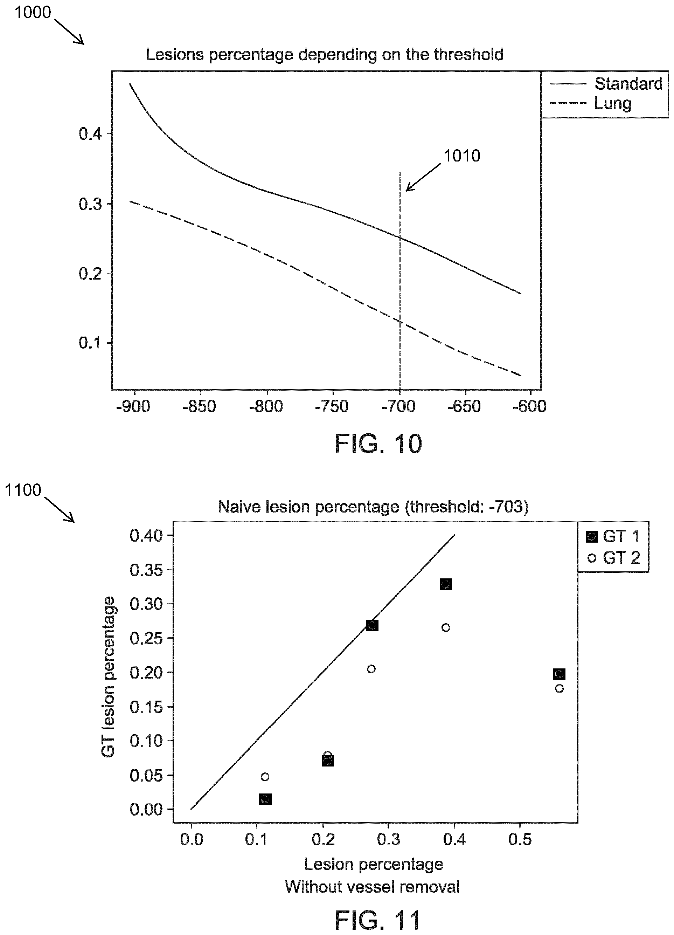

FIG. 10 is a chart illustrating lesion extent attenuation based on different reconstruction kernels. Shown in FIG. 10 is chart 1000 (x-y plane based chart) illustrating impacts of use of different reconstruction algorithms in CT scan based operations on HU based measurements. In this regard, the x-axis in chart 1000 represents the HU thresholds while the y-axis represents the lesion extent attenuation percentage. As illustrated in FIG. 10, use of lung reconstruction kernel may results in enhanced lesion measurements compared to standard reconstruction kernel for same threshold values. Thus, for an example threshold 1010 (e.g., corresponding to threshold value of -703), use of lung reconstruction kernel results in reduced lesion extent attenuation percentage compared to use of standard reconstruction kernel at that same threshold (from ~0.3 to ~1.5).

FIG. 11 is a chart illustrating impact of vessel subtraction on lesion detection. Shown in FIG. 11 is chart 1100 (x-y plane based chart) illustrating impact of vessel subtraction. In this regard, the x-axis in chart 1100 represents the lesion percentage while the y-axis represents GT lesion percentage. The data shown in chart 1100 comprise example data obtained based on study on 5 cases, with lesion extent measured manually by 2 radiologists, and compared to the volume of high densities (HU threshold > -703) on the lung segmented area. As illustrated in chart 1100, lesion extent tends to be over-estimated if vessels are not excluded.

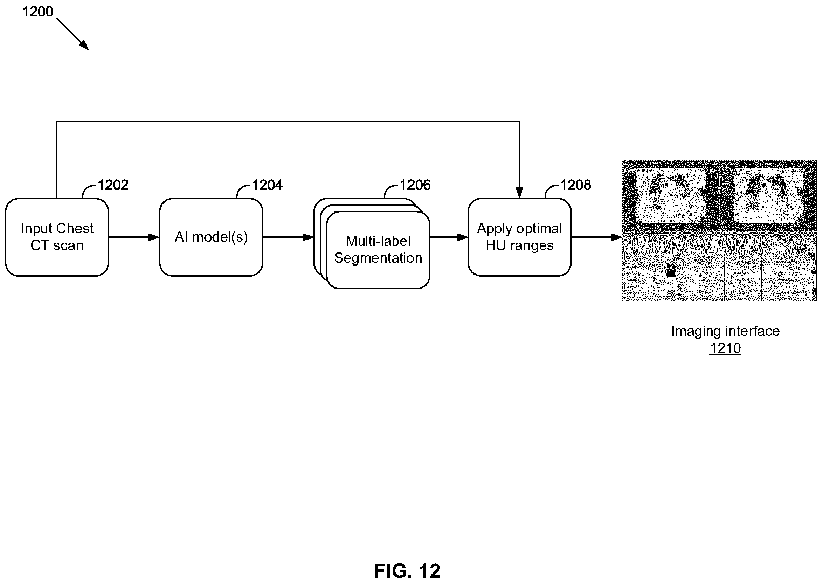

FIG. 12 is a block diagram illustrating an example flowchart of pipeline based process for use of robust intensity ranges for automatic lesion volume measurement during medical imaging operations. Shown in FIG. 12 is flowchart 1200 corresponding to pipeline based process that may be performed in a suitable system (e.g., medical imaging system 110 of FIG. 1, CT system 200 of FIG. 2) for robust intensity ranges for automatic lesion volume measurement.

In block 1202, three-dimensional (3D) CT acquisition volume(s) may be generated/captured via CT system (e.g., medical imaging system 110 of FIG. 1, CT system 200 of FIG. 2), as described above.

In block 1204, AI model(s) for use in automatic lesion volume measurement may be generated and/or trained using the 3D CT acquisition volume(s). The AI model(s) may be generated and/or trained on various types of acquisitions, patient postures, respiratory phases, etc. For example, the AI model(s) may be generated and trained using processing resources of the imaging system or a dedicated suitable system (e.g., computing system 120 of FIG. 1). In some implementations, CT acquisition datasets may be collected from various imaging systems, and used in generating and training the AI models. Once generated and/or trained, the AI models may be deployed and pre-stored into CT based systems for use therein. In some instances, the AI model training may be adjusted and/or modified, such as based on user feedback (either direct feedback, or as determined based on user's actions in response to the automatic settings).

In block 1206, multi-label segmentation may be applied to CT acquisition volume during CT based examination, particularly based on training of the system using the AI model(s). For example, in lung examinations the segmentation may be done based on and/or be directed to such features or structures as healthy tissue, pulmonary vessels, trachea and bronchi, lesion ground-glass opacity (GGO), lesion consolidation, etc.

In block 1208, best HU ranges may be selected and/or used, which allow for non-lung structure removal and auto-fitting of HU ranges. Consequently, image(s) 1210 may generated with robust intensity ranges, thus allowing for enhanced (e.g., accurate and reproducible) measurement of lesion extent.

An example method in accordance with present disclosure comprises processing imaging data obtained during medical imaging examination of a patient, wherein the imaging data correspond to a particular medical imaging technique, automatically determining one or more parameters pertinent to identifying particular features in at least one organ or body structure, generating at least one medical image based on processing of the imaging data, displaying the at least one medical image, the displaying comprises identifying, based on the one or more parameters, one or more areas in the at least one organ or body structure, and providing within displayed at least one medical image visual feedback relating to the identified one or more areas in the at least one organ or body structure.

In an example implementation, the method further comprises automatically determining the one or more parameters based on pre-trained identification model.

In an example implementation, the pre-trained identification model comprises artificial intelligence (AI) based model.

In an example implementation, the method further comprises identifying the one or more areas comprises performing density related measurements based on the one or more parameters.

In an example implementation, the at least one organ or body structure comprises at least one or both lungs.

In an example implementation, the particular features comprise lesions.

In an example implementation, the one or more parameters comprise Hounsfield scale unit (HU) thresholds.

An example non-transitory computer readable medium in accordance with the present disclosure has stored thereon a computer program having at least one code section, the at least one code section being executable by a machine comprising at least one processor, for causing the machine to perform one or more steps comprising processing imaging data obtained during medical imaging examination of a patient, wherein the imaging data correspond to a particular medical imaging technique, automatically determining one or more parameters pertinent to identifying particular features in at least one organ or body structure, generating at least one medical image based on processing of the imaging data, displaying the at least one medical image, the displaying comprises identifying, based on the one or more parameters, one or more areas in the at least one organ or body structure, and providing within displayed at least one medical image visual feedback relating to the identified one or more areas in the at least one organ or body structure.

In an example implementation, the one or more steps further comprise automatically determining the one or more parameters based on pre-trained identification model.

In an example implementation, the pre-trained identification model comprises artificial intelligence (AI) based model.

In an example implementation, the one or more steps further comprise identifying the one or more areas comprises performing density related measurements based on the one or more parameters.

In an example implementation, the at least one organ or body structure comprises at least one or both lungs.

In an example implementation, the particular features comprise lesions.

In an example implementation, the one or more parameters comprise Hounsfield scale unit (HU) thresholds.

An example system in accordance with the present disclosure comprises one or more processing circuits and a display device, with the one or more processing circuits configured to process imaging data obtained during medical imaging examination of a patient, wherein the imaging data correspond to a particular medical imaging technique, automatically determine one or more parameters pertinent to identifying particular features in at least one organ or body structure, generate at least one medical image based on processing of the imaging data, display via the display device the at least one medical image, the displaying comprises identifying, based on the one or more parameters, one or more areas in the at least one organ or body structure, and providing within displayed at least one medical image visual feedback relating to the identified one or more areas in the at least one organ or body structure.

In an example implementation, the one or more processing circuits are configured to automatically determine the one or more parameters based on pre-trained identification model.

In an example implementation, the one or more processing circuits are configured to identify the one or more areas comprises performing density related measurements based on the one or more parameters.

In an example implementation, the particular features comprise lesions and the one or more processing circuits are configured to obtain lesion extent measurements based on the one or more parameters.

In an example implementation, the one or more parameters comprise Hounsfield scale unit (HU) thresholds, and wherein the one or more processing circuits are configured to extent density-based measurements based on the HU thresholds.

As utilized herein the terms "circuits" and "circuitry" refer to physical electronic components (e.g., hardware) and any software and/or firmware ("code") which may configure the hardware, be executed by the hardware, and or otherwise be associated with the hardware. As used herein, for example, a particular processor and memory may comprise a first "circuit" when executing a first one or more lines of code and may comprise a second "circuit" when executing a second one or more lines of code. As utilized herein, "and/or" means any one or more of the items in the list joined by "and/or". As an example, "x and/or y" means any element of the three-element set {(x), (y), (x, y)}. In other words, "x and/or y" means "one or both of x and y." As another example, "x, y, and/or z" means any element of the seven-element set {(x), (y), (z), (x, y), (x, z), (y, z), (x, y, z)}. In other words, "x, y and/or z" means "one or more of x, y, and z." As utilized herein, the terms "block" and "module" refer to functions than can be performed by one or more circuits. As utilized herein, the term "exemplary" means serving as a non-limiting example, instance, or illustration. As utilized herein, the terms "for example" and "e.g.," set off lists of one or more non-limiting examples, instances, or illustrations. As utilized herein, circuitry is "operable" to perform a function whenever the circuitry comprises the necessary hardware (and code, if any is necessary) to perform the function, regardless of whether performance of the function is disabled or not enabled (e.g., by some user-configurable setting, a factory trim, etc.).

Other embodiments of the invention may provide a non-transitory computer readable medium and/or storage medium, and/or a non-transitory machine readable medium and/or storage medium, having stored thereon, a machine code and/or a computer program having at least one code section executable by a machine and/or a computer, thereby causing the machine and/or computer to perform the processes as described herein.

Accordingly, the present disclosure may be realized in hardware, software, or a combination of hardware and software. The present invention may be realized in a centralized fashion in at least one computing system, or in a distributed fashion where different elements are spread across several interconnected computing systems. Any kind of computing system or other apparatus adapted for carrying out the methods described herein is suited. A typical combination of hardware and software may be a general-purpose computing system with a program or other code that, when being loaded and executed, controls the computing system such that it carries out the methods described herein. Another typical implementation may comprise an application specific integrated circuit or chip.

Various embodiments in accordance with the present disclosure may also be embedded in a computer program product, which comprises all the features enabling the implementation of the methods described herein, and which when loaded in a computer system is able to carry out these methods. Computer program in the present context means any expression, in any language, code or notation, of a set of instructions intended to cause a system having an information processing capability to perform a particular function either directly or after either or both of the following: a) conversion to another language, code or notation; b) reproduction in a different material form.

While the present invention has been described with reference to certain embodiments, it will be understood by those skilled in the art that various changes may be made and equivalents may be substituted without departing from the scope of the present invention. In addition, many modifications may be made to adapt a particular situation or material to the teachings of the present invention without departing from its scope. Therefore, it is intended that the present invention not be limited to the particular embodiment disclosed, but that the present invention will include all embodiments falling within the scope of the appended claims.

## Claims

1. A method comprising:
processing imaging data obtained during medical imaging examination of a patient, wherein the imaging data correspond to a particular medical imaging technique;
automatically determining one or more parameters pertinent to identifying particular features in at least one organ or body structure;
generating at least one medical image based on processing of the imaging data;
displaying the at least one medical image, the displaying comprises:
identifying, based on the one or more parameters, one or more areas in the at least one organ or body structure; and
providing within displayed at least one medical image visual feedback relating to the identified one or more areas in the at least one organ or body structure.

2. The method of claim 1, automatically determining the one or more parameters based on pre-trained identification model.

3. The method of claim 2, wherein the pre-trained identification model comprises artificial intelligence (AI) based model.

4. The method of claim 1, wherein identifying the one or more areas comprises performing density related measurements based on the one or more parameters.

5. The method of claim 1, wherein the at least one organ or body structure comprises at least one or both lungs, and wherein the particular features comprise lesions.

6. The method of claim 1, wherein the one or more parameters comprise Hounsfield scale unit (HU) thresholds.

7. The method of claim 1, comprising providing, during the displaying the at least one medical image, output relating to the one or more parameters.

8. The method of claim 1, comprising enabling, during the displaying the at least one medical image, adjustments by a user to the one or more parameters.

9. A non-transitory computer readable medium having stored thereon a computer program having at least one code section, the at least one code section being executable by a machine comprising at least one processor, for causing the machine to perform one or more steps comprising:
processing imaging data obtained during medical imaging examination of a patient, wherein the imaging data correspond to a particular medical imaging technique;
automatically determining one or more parameters pertinent to identifying particular features in at least one organ or body structure;
generating at least one medical image based on processing of the imaging data;
displaying the at least one medical image, the displaying comprises:
identifying, based on the one or more parameters, one or more areas in the at least one organ or body structure; and
providing within displayed at least one medical image visual feedback relating to the identified one or more areas in the at least one organ or body structure.

10. The non-transitory computer readable medium of claim 9, wherein the one or more steps further comprise automatically determining the one or more parameters based on pre-trained identification model.

11. The non-transitory computer readable medium of claim 10, wherein the pre-trained identification model comprises artificial intelligence (AI) based model.

12. The non-transitory computer readable medium of claim 9, wherein identifying the one or more areas comprises performing density related measurements based on the one or more parameters.

13. The non-transitory computer readable medium of claim 9, wherein the at least one organ or body structure comprises at least one or both lungs, and wherein the particular features comprise lesions.

14. The non-transitory computer readable medium of claim 9, wherein the one or more steps further comprise enabling, during the displaying the at least one medical image, adjustments by a user to the one or more parameters.

15. A system comprising:
one or more processing circuits; and
a display device;
wherein the one or more processing circuits are configured to:
process imaging data obtained during medical imaging examination of a patient, wherein the imaging data correspond to a particular medical imaging technique;
automatically determine one or more parameters pertinent to identifying particular features in at least one organ or body structure;
generate at least one medical image based on processing of the imaging data;
display via the display device the at least one medical image, the displaying comprises:
identifying, based on the one or more parameters, one or more areas in the at least one organ or body structure; and
providing within displayed at least one medical image visual feedback relating to the identified one or more areas in the at least one organ or body structure.
